# EUROPEAN PATENT SPECIFICATION

(11) **EP 1 613 246 B1**
(45) Date of publication and mention of the grant of the patent: **26.09.2018**
(21) Application number: 04759372.8
(22) Date of filing: 09.04.2004
(51) Int. Cl.: A61F 2/06, A61F 2/95, A61F 2/91, A61F 2/958, A61F 2/966

(54) **MEDICAL DEVICE DELIVERY SYSTEMS**
ABGABESYSTEME FÜR MEDIZINISCHE VORRICHTUNGEN
SYSTEMES DE MISE EN PLACE DE DISPOSITIFS MEDICAUX

(30) Priority: 11.04.2003 US 411645
(43) Date of publication of application: 11.01.2006
(73) Proprietor: Boston Scientific Limited, Hamilton HM11 (BM)
(72) Inventor: GUNDERSON, Richard, C., Maple Grove, MN 55311 (US); MALEWICZ, Andrzej, M., Minneapolis, MN 55409 (US); LARSON, Karen, Lino Lakes, MN 55014 (US); MOBERG, Jonathan, R., Elk River, MN 55330 (US); SHELSO, Susan, I., Plymouth, MN 55441 (US)
(74) Representative: Vossius & Partner Patentanwälte Rechtsanwälte mbB
(86) International application number: PCT/US2004/011023
(87) International publication number: WO 2004/091446

(56) References cited:
- EP-A- 1 157 673
- WO-A-00/71058
- WO-A-02/41805
- US-A1- 2002 055 767
- US-A1- 2002 120 323
- US-A1- 2002 151 955

## Description

### TECHNICAL FIELD

This invention relates to medical device delivery systems, and related methods and components.

### BACKGROUND

Systems are known for delivering medical devices, such as stents, into a body lumen. Often, such systems include a proximal portion that remains outside the body during use and a distal portion that is disposed within the body during use. The proximal portion typically includes a handle that is held by an operator of the system (e.g., a physician) during use, and the distal portion can include a sheath surrounding a catheter with a stent positioned therebetween. Generally, the operator of the system positions the distal portion within the lumen at a desired location (e.g., so that the stent is adjacent an occlusion). The operator can then retract the sheath to allow the stent to engage the occlusion/lumen wall. Thereafter, the operator removes the distal portion of the system from the lumen. WO0241805 discloses a system for deploying a self-expandable stent comprising movement preventing elements for preventing the axial movement of the stent with relation to the catheter during retraction of the sheath for enabling an accurate deployment. WO0232496 discloses an attachment/detachment mechanism including a cylindrical lock receiving section.

The present invention defines a bumper to be included in the system to aid a stent to be deployed from it.

### SUMMARY

The invention relates to medical device delivery systems, and methods to position a stent thereon, as set fourth in the claims.

The invention defines a medical implantable endoprosthesis including an implantable medical endoprosthesis bumper with a retainer that is configured to interdigitate with a cell of an implantable medical endoprosthesis to preclude the implantable medical endoprosthesis from moving distally relative to the bumper i.e. to unidirectionally position the implantable medical endoprosthesis with respect to the bumper.

The implantable medical endoprosthesis delivery system further includes a catheter and a sheath at least partially surrounding the catheter.

The catheter and the sheath are configured so that the implantable medical endoprosthesis can be disposed between them.

In another aspect, the invention features a method of positioning an implantable medical endoprosthesis on the catheter.

The method including interdigitating a cell of the implantable medical endoprosthesis with a retainer of the bumper without reducing the diameter of the implantable medical endoprosthesis.

In one aspect, the method includes pushing the implantable medical endoprosthesis in a longitudinal or axial direction of the catheter to interdigitate a cell of the implantable medical endoprosthesis against the bumper retainer without reducing the diameter of the implantable medical endoprosthesis.

In this first aspect, the cell is a partially open cell.

In a second aspect, the invention features a method of positioning an implantable medical endoprosthesis on a catheter, the method including expanding the bumper retainer to interdigitate with a cell of the implantable medical endoprosthesis.

As mentioned above, the retainer of the implantable medical endoprosthesis delivery system of the present invention is configured to interdigitate with a cell of an implantable medical endoprosthesis with the bumper retainer to unidirectionally position the implantable medical endoprosthesis with respect to the catheter body without reducing the diameter of the implantable medical endoprosthesis. The catheter and the sheath are configured so that the implantable medical endoprosthesis can be disposed between them.

In one aspect of the invention, the cells include a partially open cell having two paddles that is located at the first end of the implantable medical endoprosthesis. The paddles of the partially open cell flex to extend around the bumper retainer.

Embodiments can include one or more of the following advantages.

In some embodiments, the implantable medical endoprosthesis delivery system can exhibit relatively high accuracy in positioning an implantable medical endoprosthesis, and/or increased control over the deployment of an implantable medical endoprosthesis.

In the invention, an implantable medical endoprosthesis can be positioned within an implantable medical endoprosthesis delivery system without reducing the diameter of the implantable medical endoprosthesis. This can increase the efficiency and/or accuracy of positioning the implantable medical endoprosthesis within the implantable medical endoprosthesis delivery system.

For example, the retainer can provide an operator of an implantable medical endoprosthesis delivery system with information about the location of an implantable medical endoprosthesis within the system. If the retainer is interdigitated with a cell of the implantable medical endoprosthesis, then the location of the implantable medical endoprosthesis should be near the location of the retainer.

Features and advantages of the invention are in the description, drawings, and claims.

### DESCRIPTION OF DRAWINGS

FIGS. 1A-1C illustrate a process for attaching an embodiment of a stent to an embodiment of a stent bumper.
FIGS. 1D-1F illustrate the deployment of the stent of FIGS. 1A-1C.
FIG. 1G is a perspective view of the stent bumper of FIGS. 1A-1F.
FIG. 2 is a perspective view of an embodiment of a stent bumper.
FIG. 3 is a perspective view of an embodiment of a stent bumper.
FIGS. 4A and 4B illustrate a process for attaching an embodiment of a stent to an embodiment of a stent bumper.
FIG. 5 is a perspective view of an embodiment of a stent bumper.
FIG. 6 is a perspective view of an embodiment of a stent bumper.
FIGS. 7A-7C illustrate a process for attaching an embodiment of a stent to the stent bumper of FIG. 6.
FIGS. 8A-8G illustrate a process for attaching an embodiment of a stent to an embodiment of a stent bumper.
FIG. 9 is a perspective view of an embodiment of a stent bumper.
FIG. 10 is a perspective view of an embodiment of a stent bumper.
FIG. 11 is a perspective view of an embodiment of a stent bumper.
FIG. 12 is a perspective view of an example of a stent bumper.
FIGS. 13A-13C illustrate a process for attaching a stent to the stent bumper of FIG. 12.
FIG. 14 is a perspective view of an example of a stent bumper.
FIG. 15 is a side view of an embodiment of a stent bumper.
FIG 16 is a side view of an example of a stent bumper.
FIG. 17 is a side view of an example of a stent bumper.
FIG. 18 is a side view of an example of a stent bumper.
FIG. 19 is a side view of an example of a stent bumper.
FIGS. 20A and 20B illustrate a process for attaching an embodiment of a stent to an embodiment of a stent bumper.
FIG. 21 is a front view of an example of a stent bumper.
FIGS. 22A and 22B illustrate a process for attaching a stent to the stent bumper of FIG. 21.

### DETAILED DESCRIPTION

In general, the delivery systems are designed to temporarily retain an implantable medical endoprosthesis during delivery to a target site by interdigitating a retainer with a cell of the endoprosthesis so that the endoprosthesis does not prematurely deploy from the delivery device. It is believed that such systems can increase the accuracy of positioning the implantable medical endoprosthesis within a subject. Certain embodiments of the systems are disclosed below.

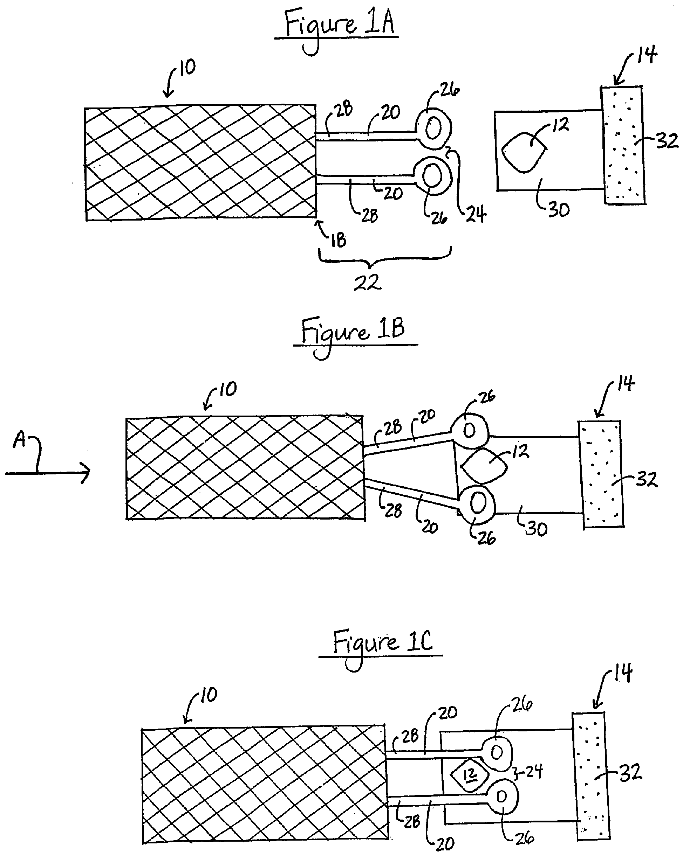

For example, FIGS. 1A-1C show the interdigitation of a partially open cell 22 of a self-expanding stent 10 with a bumper retainer 12 of a bumper 14. Stent 10 has an end 18 that includes partially open cell 22, which has two paddles 20, each with respective heads 26 and legs 28. With this arrangement, partially open cell 22 can interdigitate with stent retainer 12 so that stent 10 is unidirectionally positioned with respect to bumper 14 (see discussion below). Paddles 20, which are formed of a flexible material, can be used, for example, to incorporate radiopaque material (e.g., radiopaque markers) that renders stent 10 radiopaque. Partially open cell 22 has an opening 24 that is smaller than bumper retainer 12. When paddles 20 are pushed against bumper retainer 12 in the direction of arrow A, as shown in FIG. 1B, paddles 20 flex to extend around bumper retainer 12. As paddles 20 flex, the size of opening 24 temporarily increases, such that bumper retainer 12 is accommodated by opening 24. As FIG. 1C shows, once heads 26 of paddles 20 have passed bumper retainer 12, paddles 20 return to their original position. In doing so, paddles 20 lock bumper retainer 12 between legs 28, thereby interdigitating partially open cell 22 with bumper retainer 12. The flexing of paddles 20 around bumper retainer 12 allows for interdigitation between partially open cell 22 and retainer 12 without a reduction in the diameter of stent 10.

The interdigitation of partially open cell 22 of stent 10 with bumper retainer 12 precludes stent 10 from moving distally relative to bumper 14 as the sheath of a stent delivery system is withdrawn over stent 10 during delivery of stent 10 to a target site (i.e., stent retainer 12 unidirectionally positions stent 10 relative to bumper 14).

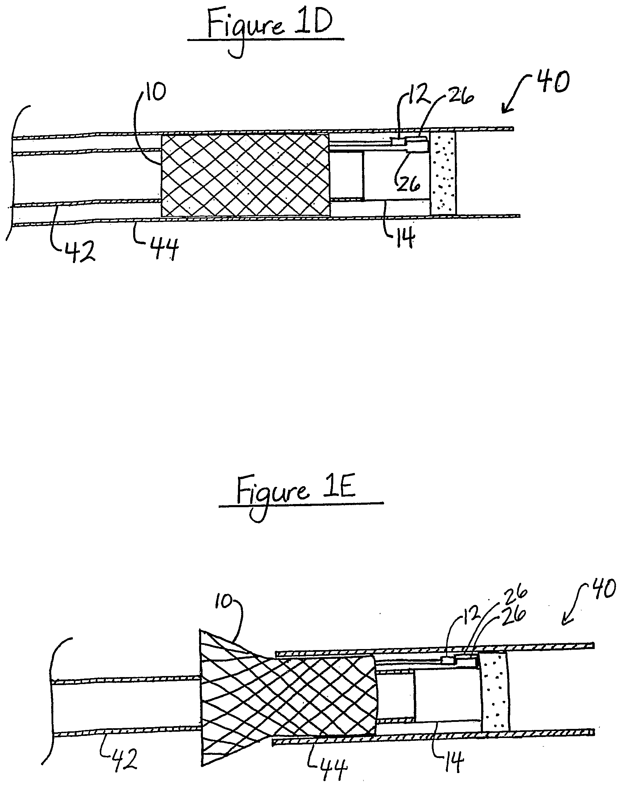

FIGS. 1D-1F illustrate the delivery of stent 10 to a target site using a delivery device 40. Delivery device 40 includes an inner catheter 42 and a sheath 44. Bumper 14 is loaded onto inner catheter 42, and is located between inner catheter 42 and sheath 44. Stent 10 is positioned on inner catheter 42, and is disposed between inner catheter 42 and sheath 44. Bumper retainer 12 prevents stent 10 from deploying prematurely (e.g., before sheath 44 is substantially retracted) by interdigitating with partially open cell 22.

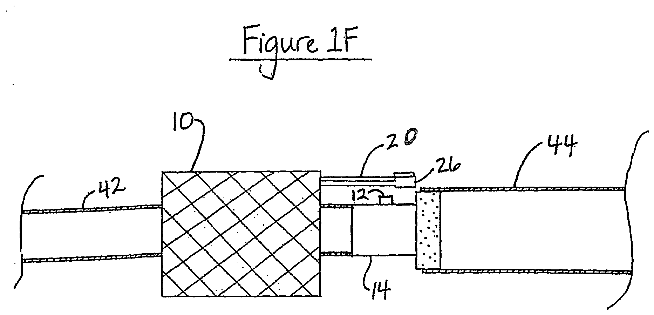

FIGS. 1E and 1F show the deployment of stent 10. As sheath 44 is retracted, stent 10 begins to expand, lifting paddles 20 away from bumper retainer 12. Once sheath 44 has been retracted past paddles 20, bumper retainer 12 no longer interdigitates with partially open cell 22. Inner catheter 42 can then be proximally withdrawn while leaving stent 10 at the target site.

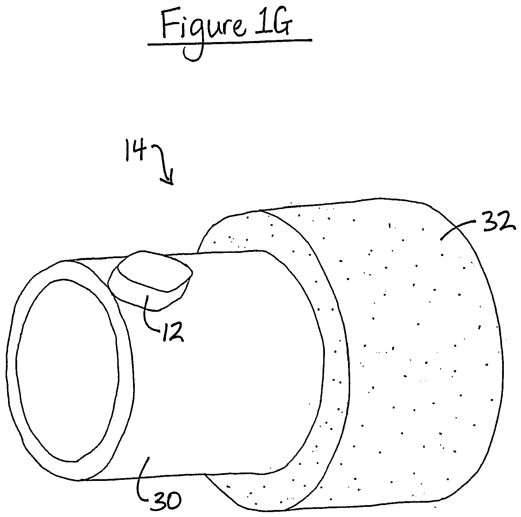

FIG. 1G shows bumper 14 in enlarged detail. Bumper 14 includes a body portion 30 and a head portion 32 that is integral with body portion 30. Bumper retainer 12, which is diamond-shaped, protrudes radially from the surface of body portion 30. Bumper retainer 12 can be integrally formed (e.g., molded) with body portion 30, or can be formed separately from body portion 30 and then attached (e.g., adhesive-bonded) to body portion 30.

Bumper retainer 12 can be formed of, for example, a polymer, a metal, an alloy or a combination of these materials. Examples of polymers include polyether-block co-polyamide polymers (e.g., PEBAX®), copolyester elastomers (e.g., Arnitel® copolyester elastomers), thermoplastic polyester elastomers (e.g., Hytrel®), thermoplastic polyurethane elastomers (e.g., Pellethane™), polyeolefins (e.g., Marlex® polyethylene, Marlex® polypropylene), high-density polyethylene (HDPE), low-density polyethylene (LDPE), polyamides (e.g., Vestamid®), and combinations of these materials. Examples of metals and alloys include stainless steel, platinum, gold, tantalum, and MP35N (a nickel-cobalt-chromium-molybdenum alloy). In some embodiments, bumper retainer 12 and body portion 30 and/or head portion 32 can be formed of the same materials. In certain embodiments, bumper retainer 12 and body portion 30 and/or head portion 32 can be formed of different materials.

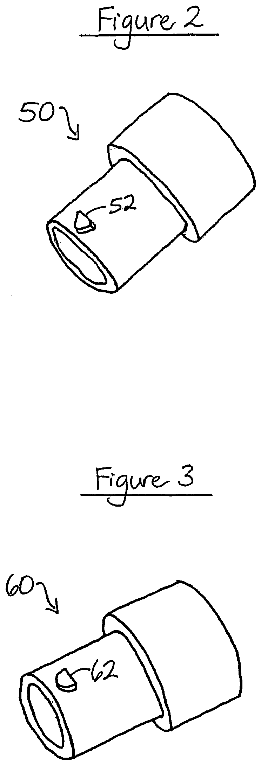

While a diamond-shaped bumper retainer is shown in FIGS. 1A-1G, bumper retainers can have other shapes. In general, bumper retainers can be shaped to unidirectionally position an implantable medical endoprosthesis with respect to a bumper. For example, FIG. 2 shows a bumper 50 with a triangular bumper retainer 52, and FIG. 3 shows a bumper 60 with a hemispherical bumper retainer 62.

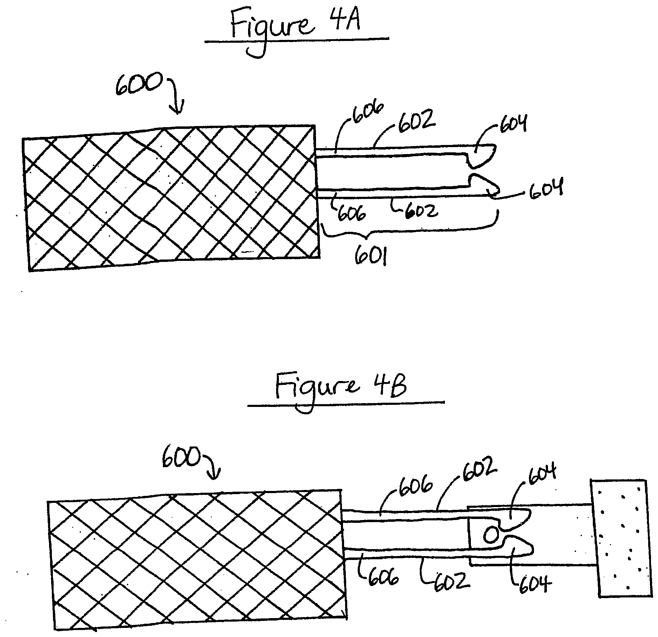

In some embodiments, a bumper can include a nodular bumper retainer. In such embodiments, a stent that interdigitates with the bumper retainer can have a partially open cell that is designed to have a reduced likelihood of becoming prematurely disengaged from the bumper retainer. For example, FIG. 4A shows a stent 600 with a partially open cell 601 having two paddles 602, each of which has a triangular head 604 and a leg 606. As shown in FIG. 4B, after paddles 602 have flexed around a nodular bumper retainer 608 of a bumper 610, heads 604 of paddles 602 temporarily lock around bumper retainer 608. The shape of triangular heads 604 helps to limit the likelihood that partially open cell 601 will prematurely disengage from bumper retainer 608.

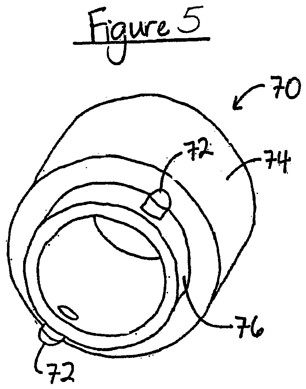

In some embodiments, a bumper can include more than one bumper retainer. For example, FIG. 5 shows a bumper 70 with a head portion 74, a body portion 76, and two oppositely disposed nodular bumper retainers 72, which protrude radially from body portion 76. While two bumper retainers are shown in FIG. 5, in some embodiments, a bumper can include more than two (e.g., three, four, five, six, seven, eight, nine, ten) bumper retainers. As the number of bumper retainers on a bumper increases, the retention of an endoprosthesis can be enhanced, and the accuracy of endoprosthesis positioning can increase. In certain embodiments, the number of bumper retainers on a bumper can correspond to the number of partially open cells in a stent with which the bumper is interdigitating. The bumper retainers can be disposed equidistantly from each other on the bumper, or can be disposed at varying distances from each other. The bumper retainers can have the same shapes or different shapes.

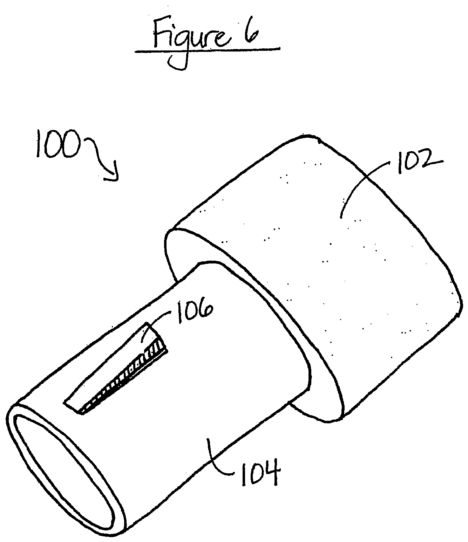

While certain shapes of bumper retainers have been shown, other shapes may also be used. For example, a bumper retainer can be a flap, a hook, or a barb or spike. As an example, FIG. 6 shows a bumper 100 with a head portion 102 and a body portion 104. A bumper retainer 106 in the form of a rectangular-shaped flap projects radially from body portion 104.

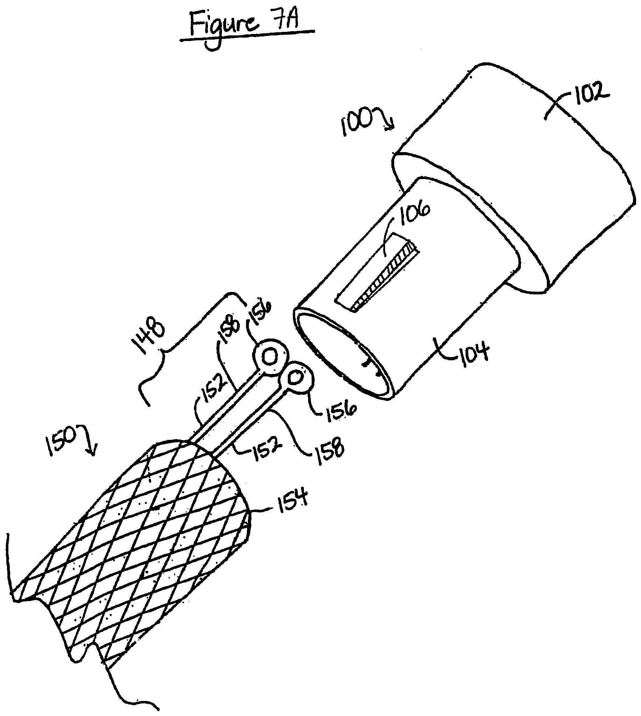

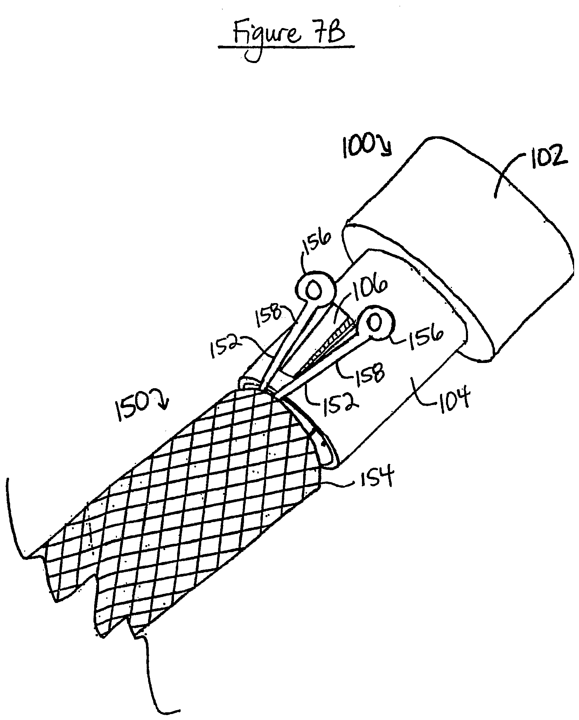

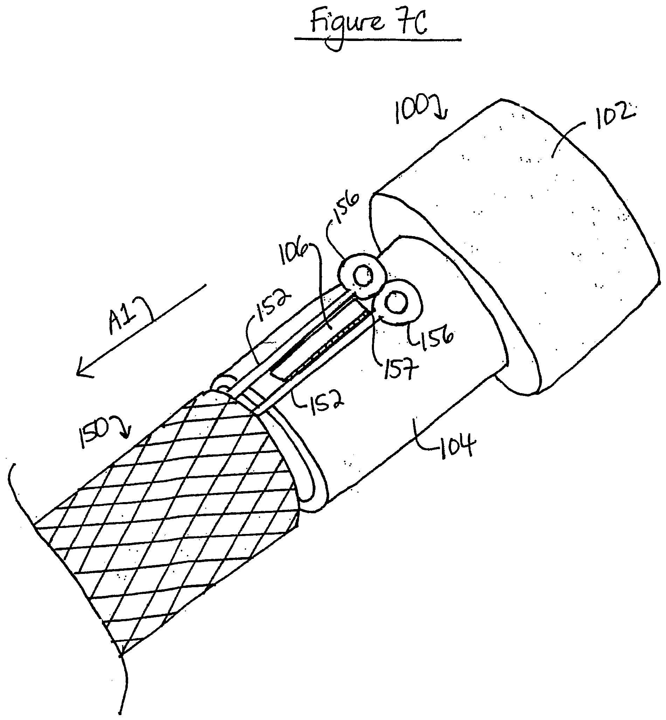

FIGS. 7A-7C show the interdigitation of bumper retainer 106 with a partially open cell 148 of a stent 150. As shown in FIG. 7A, stent 150 has an end 154 that includes partially open cell 148, which has two flexible paddles 152, each with respective heads 156 and legs 158. As FIG. 7B shows, when stent 150 is pushed toward bumper retainer 106, paddles 152 flex outwardly to fit around bumper retainer 106. After heads 156 of paddles 152 have flexed around bumper retainer 106, stent 150 is pulled back in the direction of arrow A1, such that heads 156 contact the proximal end 157 of bumper retainer 106, thereby interdigitating bumper retainer 106 with partially open cell 148. As stent 150 is delivered to a target site and expanded, paddles 152 lift away from bumper retainer 106, thereby disengaging from partially open cell 148.

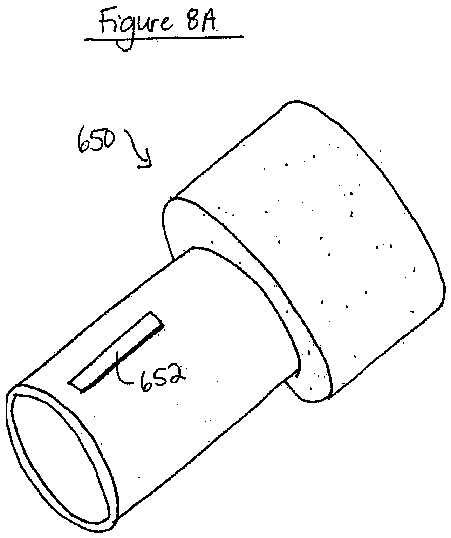

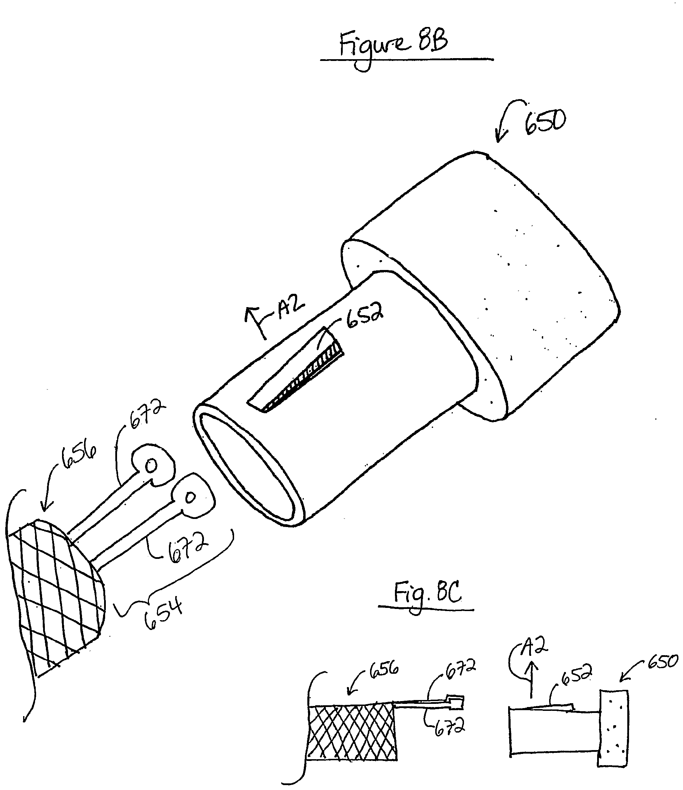

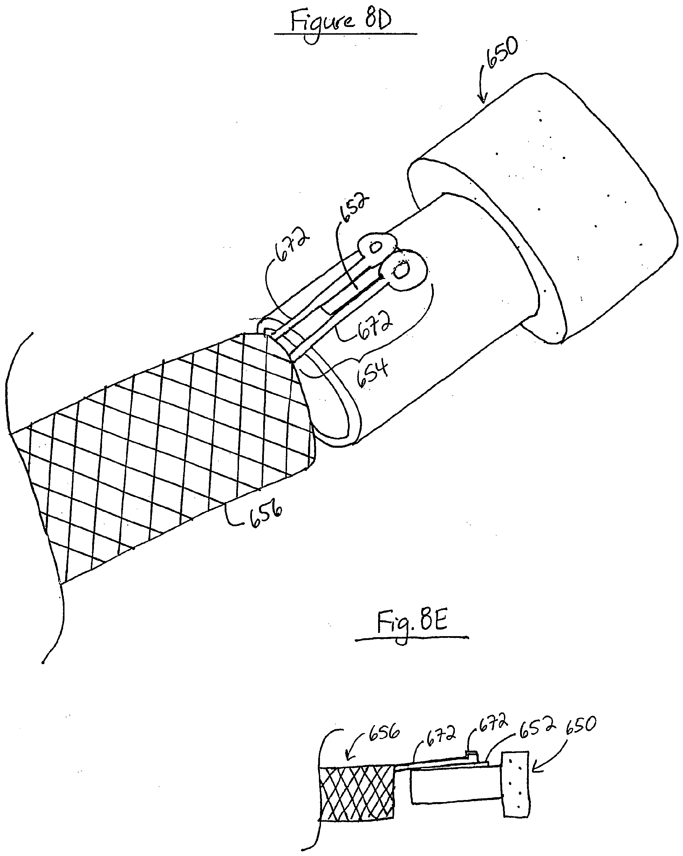

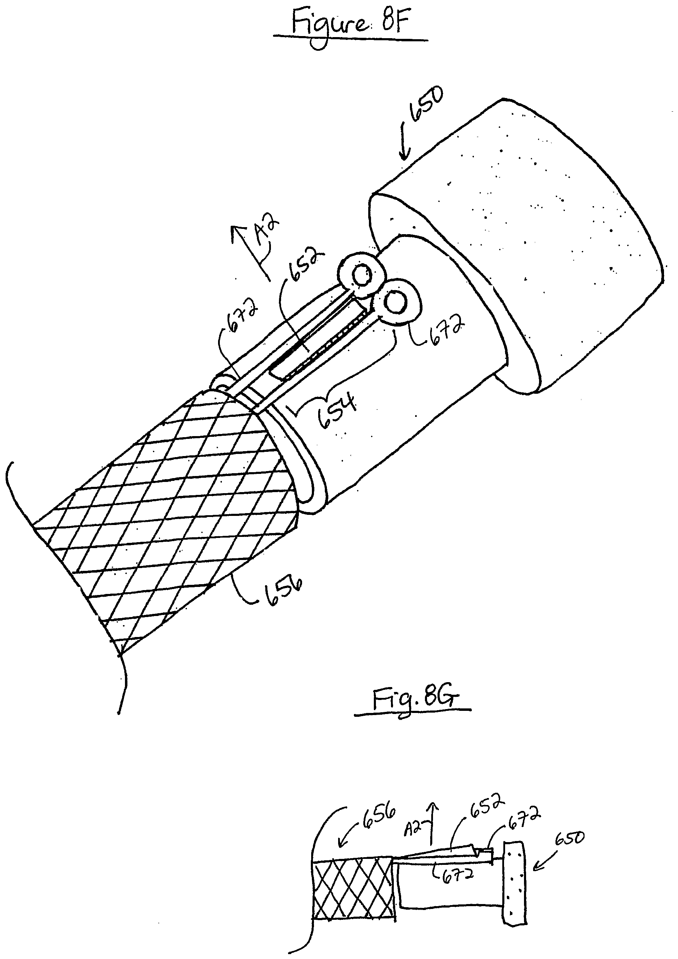

While interdigitation by paddle flexing has been shown, in some embodiments, a flap-shaped bumper retainer can interdigitate with a partially open cell by flexing through the partially open cell, while the components of the partially open cell do not flex substantially. For example, FIG. 8A shows a bumper 650 with a bumper retainer 652 in the form of a rectangular flap. As FIGS. 8B and 8C show, bumper retainer 652 can flex upward (in the direction of arrow A2). However, and referring also now to FIGS. 8D and 8E, as a stent 656 with a partially open cell 654 including paddles 672 approaches and contacts bumper retainer 652, paddles 672 push down on bumper retainer 652. As shown in FIGS. 8F and 8G, once paddles 672 have passed over bumper retainer 652, bumper retainer 652 flexes back upward in the direction of arrow A2, through partially open cell 654. Thus, bumper retainer 652 interdigitates with partially open cell 654.

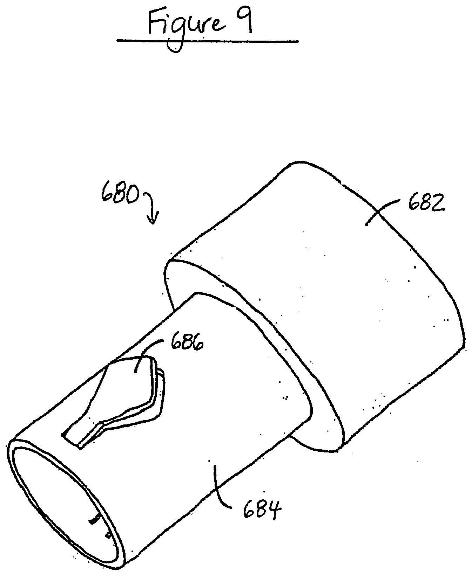

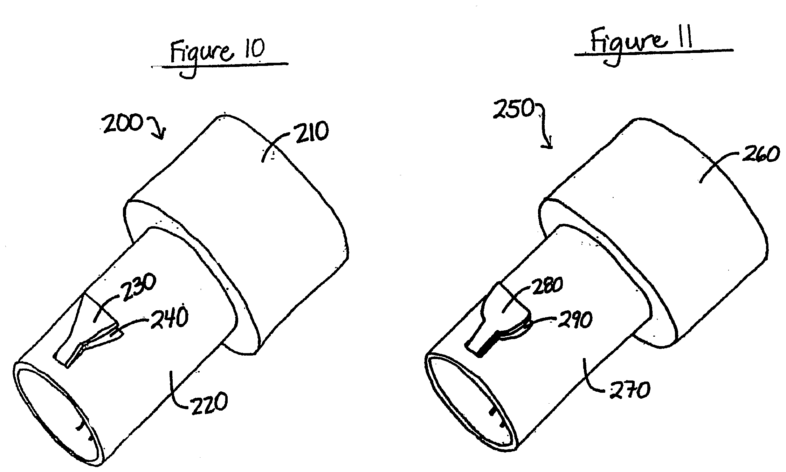

While bumper retainer 652 of FIGS. 8A-8G is a rectangular-shaped flap, flaps with other shapes can be used as bumper retainers. As an example, FIG. 9 shows a bumper 680 with a head portion 682 integrally connected to a body portion 684 that includes a bumper retainer 686. Bumper retainer 686 is in the form of a diamond-shaped flap that protrudes from body portion 684. As another example, FIG. 10 shows a bumper 200 with a head portion 210 integrally connected to a body portion 220 that includes a bumper retainer 230. Bumper retainer 230 is in the form of a triangular flap that protrudes from body portion 220. As a further example, FIG. 11 shows a bumper 250 with a head portion 260 integrally connected to a body portion 270 that includes a bumper retainer 280. Bumper retainer 280 is in the form of a hemispherical flap that protrudes from body portion 270. Another example of a suitable shape for a flap-style bumper retainer is a J-hook.

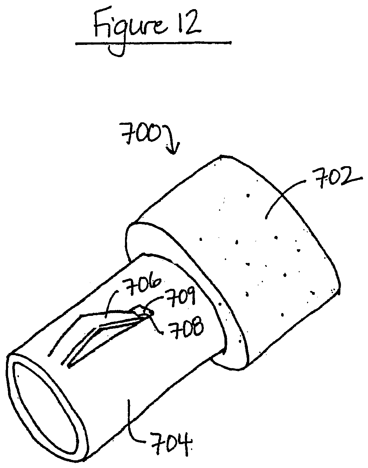

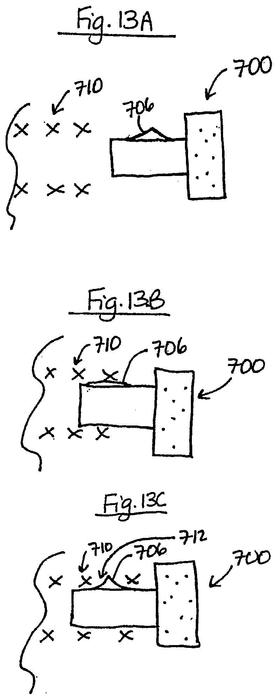

In some examples not forming part of the invention, a bumper retainer can be in the form of a flexible, bent flap which can, for example, interdigitate with a stent having a partially open cell or a stent without a partially open cell. For example, FIG. 12 shows a bumper 700 that includes a head portion 702 and a body portion 704, from which a rectangular flap-shaped bumper retainer 706 protrudes. Bumper retainer 706 is bent so that its end 708 contacts the surface 709 of body portion 704. As shown in FIGS. 13A-13C, as bumper retainer 706 comes into contact with a stent 710, bumper retainer 706 flexes in a downward direction to accommodate stent 710. However, when a cell 712 of stent 710 is disposed over bumper retainer 706, bumper retainer 706 flexes up into the cell, thereby interdigitating with the cell.

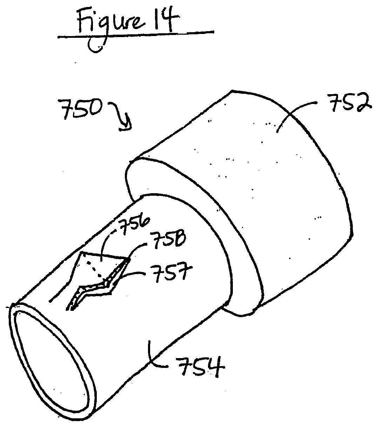

While FIGS. 12-13C show a bent rectangular flap-shaped bumper retainer, bumper retainers with other shapes can also be bent. For example, FIG. 14 shows a bumper 750 with a head portion 752 and a body portion 754 from which a flexible bumper retainer 756 in a diamond-shaped flap style protrudes. Bumper retainer 756 is bent so that end 758 of bumper retainer 756 contacts the surface 757 of body portion 754.

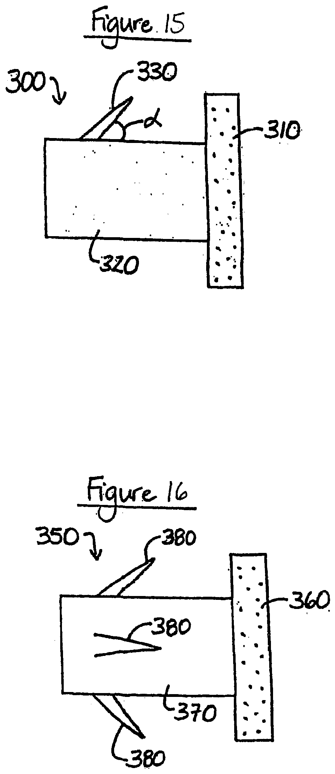

As noted above, in some embodiments, a bumper retainer can be in the form of a barb or a spike. For example, FIG. 15 shows a bumper 300 with a head portion 310 and a body portion 320, and a bumper retainer 330 protruding radially from body portion 320. Bumper retainer 330 protrudes radially from body portion 320 at an angle α selected to accommodate a partially open cell of a stent. In some embodiments, angle α can be up to about 90° (e.g., from about 30° to about 90°, up to about 10°, up to about 15°, up to about 20°).

In certain examples not forming part of the invention, a bumper can include multiple spike-shaped bumper retainers. For example, FIG. 16 shows a bumper 350 with a head portion 360 and a body portion 370, and multiple spike-shaped bumper retainers 380 protruding radially from body portion 370.

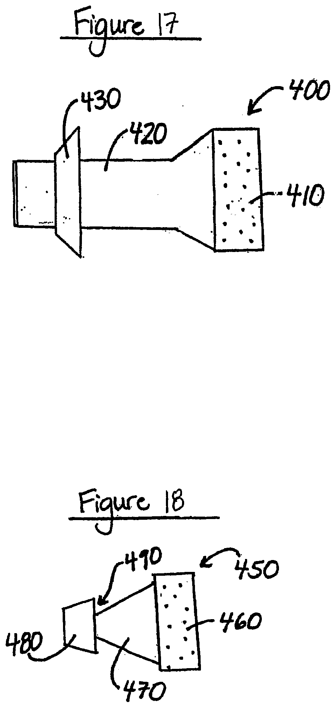

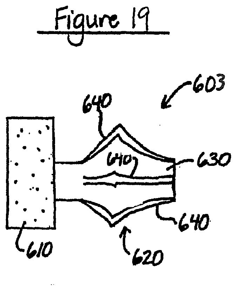

In some examples not forming part of the invention, a portion of a bumper can be molded to include one or more components that can interdigitate with a cell of a stent. For example, FIG. 17 shows a bumper 400 with a head portion 410, a body portion 420, and a collar 430 molded around body portion 420. Collar 430 functions as a bumper retainer, and is shaped to interdigitate with a cell of a stent to unidirectionally position the stent with respect to bumper 400. Similarly, FIG. 18 shows a bumper 450 with a head portion 460, a body portion 470, and a bumper retainer 480 molded to an end 490 of body portion 470. As another example, FIG. 19 shows a bumper 603 with a head portion 610 and a body portion 620 that includes a bumper retainer 630 with multiple peaks 640. In some examples, bumper retainers such as those described above can have relatively smooth surfaces to, for example, limit damage to an endoprosthesis upon interdigitation of a cell of the endoprosthesis with the bumper retainer. In certain examples, bumper retainers such as those shown in FIGS. 17-19 can be formed of a relatively soft polymer, such as a low durometer polyether-bloclc co-polyamide polymers (e.g., low durometer Pebax®), a thermoplastic resin (e.g., C-Flex®, a thermoplastic polyurethane (e.g., an aromatic polyether-based thermoplastic polyurethane such as Tecothane®)), an elastomer, or silicone. In examples in which a bumper retainer is formed of a relatively soft polymer, the bumper retainer can, for example, adjust to accommodate a stent as the stent is being disposed over the bumper retainer.

In certain embodiments, an endoprosthesis can be temporarily retained by disposing the endoprosthesis over a retainer and expanding the retainer such that it interdigitates with a cell of the endoprosthesis.

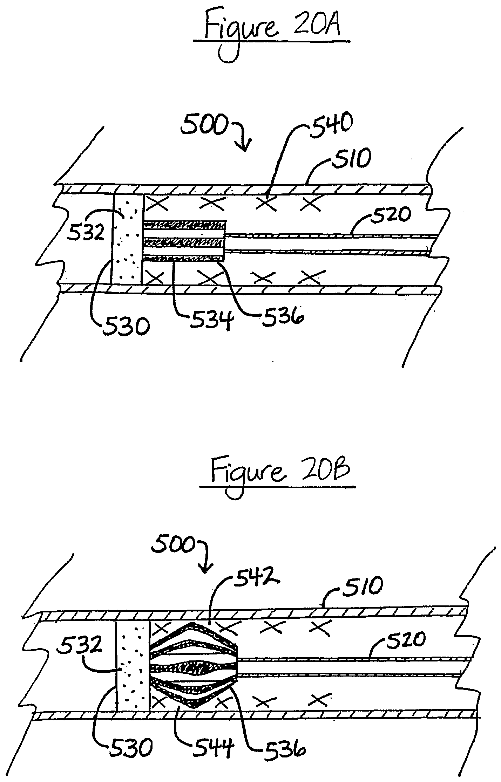

For example, FIGS. 20A and 20B show a stent delivery system 500 that includes a sheath 510, an inner catheter 520 concentrically disposed within sheath 510, and a bumper 530 attached to inner catheter 520 and restrained within sheath 510. Bumper 530 includes a head portion 532, a body portion 534, and a bumper retainer 536 on body portion 534. Bumper retainer 536 is formed of a shape-memory material. A stent 540 abuts head portion 532 of bumper 530 and is constrained between sheath 510 and inner catheter 520. FIG. 20A shows stent delivery system 500 when there is no interdigitation between any of the cells of stent 540 and bumper retainer 536. However, as FIG. 20B shows, when bumper retainer 536 is exposed to a change in temperature (e.g., when stent delivery system 500 is delivered into the body of a subject), bumper retainer 536 expands to interdigitate with cells 542 and 544 of stent 540.

As noted, bumper retainer 536 is formed of a shape-memory material. Examples of shape memory materials include metal alloys, such as nitinol (e.g., 55% nickel, 45% titanium), silver-cadmium (Ag-Cd), gold-cadmium (Au-Cd), gold-copper-zinc (Au-Cu-Zn), copper-aluminum-nickel (Cu-Al-Ni), copper-gold-zinc (Cu-Au-Zn), copper-zinc/(Cu-Zn), copper-zinc-aluminum (Cu-Zn-Al), copper-zinc-tin (Cu-Zn-Sn), copper-zinc-xenon (Cu-Zn-Xe), iron beryllium (Fe₃Be), iron platinum (Fe₃Pt), indium-thallium (In-Tl), iron-manganese (Fe-Mn), nickel-titanium-vanadium (Ni-Ti-V), iron-nickel-titanium-cobalt (Fe-Ni-Ti-Co) and copper-tin (Cu-Sn). See, e.g., Schetsky, L. McDonald, "Shape Memory Alloys", Encyclopedia of Chemical Technology (3rd ed.), John Wiley & Sons, 1982, vol. 20. pp. 726-736.

While certain embodiments have been described, other embodiments are possible.

As an example, while certain embodiments of a partially open cell have been described, other embodiments are also possible. In general, a partially open cell of an implantable medical endoprosthesis can have a configuration capable of interdigitating with a retainer (e.g., a bumper retainer) of an implantable medical endoprosthesis delivery system.

As another example, in some embodiments, a bumper retainer can be formed of a moldable material, such as a wax. One or more cells of an endoprosthesis can be interdigitated with the bumper retainer by molding the wax so that it extends into the cell(s). The cell(s) of the endoprosthesis can be subsequently disengaged from the bumper retainer when the endoprosthesis expands. Examples of waxes include medical-grade viscoelastic wax, such as dental wax (e.g., utility dental wax, dental modeling wax).

As an additional example, in certain embodiments, a bumper retainer can be formed of a swellable material, such as a hydrogel (e.g., the Tecophilic® family of hydrogels, such as Tecogel®) or a polyamide. For example, the bumper retainer can be formed of a heat-activated swellable material that expands upon exposure to an increase in temperature. In some embodiments, the bumper retainer can be formed of a material used in certain types of gaskets, such as O-rings. Examples of heat-activated swellable materials include virgin Teflon®, filled Teflon® (e.g., Teflon® including a strength-enhancing filler), expanded Teflon®, Viton®, Kalrez®, Simriz®, Chemraz®, Aflas™, fluorosilicone, urethane, hydrogenated nitrile elastomer (HNBR), polyacrylate, neoprene, and chlorosulfonated polyethylene (e.g., Hypalon®). The bumper retainer can interdigitate with a cell of an endoprosthesis by disposing the cell over the bumper retainer and allowing the bumper retainer to swell (e.g., upon exposure to a change in temperature, pH, or ion concentration) and protrude into the cell.

As a further example, while bumper retainers have been described, in some embodiments, an endoprosthesis delivery system can alternatively or additionally include one or more retainers in a different location. For example, a retainer can protrude radially from the inner catheter of an endoprosthesis delivery system. In certain embodiments, a retainer can be formed on a tubular band (e.g., a metallic band) that can, for example, be loaded onto an inner catheter and disposed between the inner catheter and an endoprosthesis.

As another example, while interdigitation with a partially open cell has been shown, in some examples, a retainer can interdigitate with a closed cell of an implantable medical endoprosthesis (a cell that is defined by a boundary that has no openings). For example, a bumper retainer can be flexed to extend through a closed cell of a stent.

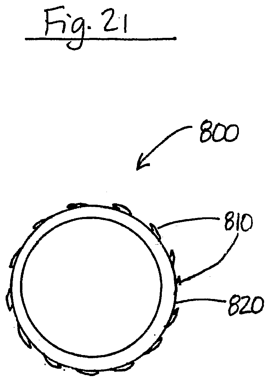

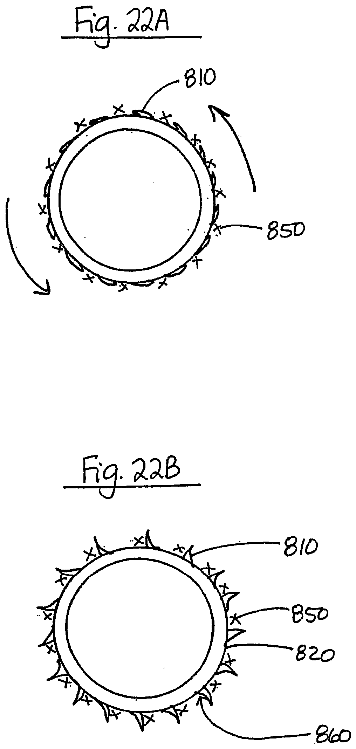

As an additional example, while interdigitation of a stent with a bumper retainer by moving the stent in a longitudinal direction or by expanding the bumper retainer have been shown, in some examples, a bumper retainer can be rotated to interdigitate with a stent. For example, FIG. 21 shows a front view of a bumper 800 with several flexible flap-style bumper retainers 810 protruding from its surface 820. As shown in FIGS. 22A and 22B, when a stent 850 is disposed over bumper 800, and bumper 800 is rotated in a counterclockwise direction, bumper retainers 810 push against stent 850, which causes bumper retainers 810 to protrude more from surface 820. As a result, bumper retainers 810 interdigitate with the cells 860 of stent 850.

Other embodiments are in the claims.

## Claims

1. A method of positioning a stent (10, 600, 150, 540, 850) on a delivery system, the delivery system comprising
an inner catheter (42, 520),
a sheath (44, 510), and
a bumper (14, 50, 60, 610, 70, 100, 680, 200, 250, 300, 350, 600, 530) loaded onto the inner catheter (42, 520), the bumper (14, 50, 60, 610, 70, 100, 680, 200, 250, 300, 350, 600, 530) including a body portion (30, 76, 104, 684, 220, 270, 320, 370, 620, 354) and a bumper retainer (12, 52, 62, 608, 72, 106, 686, 230, 280, 330, 380, 630, 536) that protrudes radially from the body portion (30, 76, 104, 684, 220, 270, 320, 370, 620, 354) for precluding the stent (10, 600, 150, 540) from moving distally as the sheath (44, 520) is withdrawn during delivery of the stent (10, 600, 150, 540), **characterized in that** the method includes the step of interdigitating a cell (22, 601, 148, 542, 544) of the stent (10, 600, 150, 540) with the bumper retainer (12, 52, 62, 608, 72, 106, 686, 230, 280, 330, 380, 630, 536) is carried out without reducing a diameter of the stent (10, 600, 150, 540) by two paddles (20, 602, 152, 672) included in the cell of the stent (10, 600, 150, 540, 850) which flex to extend around the bumper retainer (12, 52, 62, 608, 72, 106, 686, 230, 280, 330, 380, 630) when pushing the stent (10, 600, 150, ) in an axial direction (A) against the bumper retainer (12, 52, 62, 608, 72, 106, 686, 230, 280, 330, 380, 630) wherein the cell is a partially open cell, or by expanding the bumper retainer (536, 810) to interdigitate with the cell (542, 544) of the stent (540).

2. The method of claim 1, wherein the bumper retainer (12, 52, 62, 608, 72, 106, 686, 230, 280, 330, 380, 630) comprises a flap that protrudes radially from the body portion (30, 76, 104, 684, 220, 270, 320, 370, 620, 354).

3. The method of claim 1, wherein the bumper retainer (12, 52, 62, 608, 72, 106, 686, 230, 280, 330, 380, 630) comprises a hook, a spike, or a barb.

4. The method of claim 1, wherein the bumper retainer (12, 52, 62, 608, 72, 106, 686, 230, 280, 330, 380, 630) is capable of flexing to interdigitate or to disengage from the partially open cell (22, 601, 148).

5. The method of claim 1, wherein the bumper retainer (12, 52, 62, 608, 72, 106, 686, 230, 280, 330, 380, 630) comprises a wax.

6. The method of one of claims 1 to 4, wherein the two paddles (20, 602, 152, 672) each have a head (26, 604, 156) and a leg (28, 606, 158).

7. The method of claim 6, wherein the paddles (20, 602, 152, 672) are formed of a radiopaque material.

8. The method of claim 6 or 7, wherein flexing of the paddles (20, 602, 152, 672) around the bumper retainer (12, 52, 62, 608, 72, 106, 686, 230, 280, 330, 380, 630) allows for interdigitation between the partially open cell (22, 601, 148) and the retainer (12, 52, 62, 608, 72, 106, 686, 230,280, 330, 380, 630).

9. The method of claim 1, wherein the bumper retainer (536) of the catheter is configured to expand from a first size to a second size.

10. The method of claim 1 or 9, wherein the bumper retainer (536) of the catheter is formed of a shape-memory material.

11. A stent delivery system, comprising:
a catheter having an inner catheter (42, 520),
a sheath (44, 510) at least partially surrounding the catheter,
a stent (10, 600, 150, 540) disposed between the catheter and the sheath,
a bumper (14, 50, 60, 610, 70, 100, 680, 200, 250, 300, 350, 600, 530) loaded onto the inner catheter (42, 520), the bumper (14, 50, 60, 610, 70, 100, 680, 200, 250, 300, 350, 600, 530) including a body portion (30, 76, 104, 684, 220, 270, 320, 370, 620, 354) and a bumper retainer (12, 52, 62, 608, 72, 106, 686, 230, 280, 330, 380, 630, 536) that protrudes radially from the body portion (30, 76, 104, 684, 220, 270, 320, 370, 620, 354),
wherein the bumper retainer (12, 52, 62, 608, 72, 106, 686, 230, 280, 330, 380, 630, 536) is configured to interdigitate with a cell (22, 601, 148, 542, 544) of the stent (10, 600, 150, 540) to unidirectionally position the stent (10, 600, 150, 540) with respect to the inner catheter **characterized in that** the stent delivery system is configured to interdigitate a cell (22, 601, 148, 542) of the stent (10, 600, 150, 540) with the bumper retainer (12, 52, 62, 608, 72, 106, 686, 230, 280, 330, 380, 630, 536) without reducing a diameter of the stent (10, 600, 150, 540) by two paddles (20, 602, 152, 672) included in the cell of the stent (10, 600, 150, 540) which flex to extend around the bumper retainer (12, 52, 62, 608, 72, 106, 686, 230, 280, 330, 380, 630) when the stent (10, 600, 150,) is pushed in axial direction (A) against the bumper retainer (12, 52, 62, 608, 72, 106, 686, 230, 280, 330, 380, 630) wherein the cell is a partially open cell, or by an expanding bumper retainer (536) that interdigitates with a cell (542, 544) of the stent (540).

12. The stent delivery system of claim 11, wherein the bumper retainer (12, 52, 62, 608, 72, 106, 686, 230, 280, 330, 380, 630) comprises a flap that protrudes radially from the body portion (30, 76, 104, 684, 220, 270, 320, 370, 620, 354).

13. The stent delivery system of claim 12, wherein the bumper retainer (12, 52, 62, 608, 72, 106, 686, 230, 280, 330, 380, 630) comprises a hook, a spike, or a barb.

14. The stent delivery system of claim 13, wherein the bumper retainer (12, 52, 62, 608, 72, 106, 686, 230, 280, 330, 380, 630) is capable of flexing to interdigitate or to disengage from the partially open cell (22, 601, 148).

15. The stent delivery system of claim 14, wherein the bumper retainer (12, 52, 62, 608, 72, 106, 686, 230, 280, 330, 380, 630) comprises a wax.

16. The stent delivery system of one of claims 11 to 14, wherein the two paddles (20, 602, 152, 672) each have a head (26, 604, 156) and a leg (28, 606, 158).

17. The stent delivery system of claim 18, wherein the paddles (20, 602, 152, 672) are formed of a radiopaque material.

18. The stent delivery system of claim 16 or 17, wherein flexing of the paddles (20, 602, 152, 672) around the bumper retainer (12, 52, 62, 608, 72, 106, 686, 230, 280, 330, 380, 630) allows for interdigitation between the partially open cell (22, 601, 148, 712) and the bumper retainer (12, 52, 62, 608, 72, 106, 686, 230, 280, 330, 380, 630).

19. The stent delivery system of claim 11, wherein the bumper retainer (536) of the catheter is configured to expand from a first size to a second size.

20. The stent delivery system of claim 11 or 19, wherein the bumper retainer (536) of the catheter is formed of a shape-memory material.

## Patentansprüche

1. Verfahren zum Positionieren eines Stents (10, 600, 150, 540, 850) auf einem Zufuhrsystem, wobei das Zufuhrsystem aufweist:
einen inneren Katheter (42, 520);
eine Hülse (44, 510);
ein Pufferelement (14, 50, 60, 610, 70, 100, 680, 200, 250, 300, 350, 600, 530), das in den inneren Katheter (42, 520) geladen ist, wobei das Pufferelement (14, 50, 60, 610, 70, 100, 680, 200, 250, 300, 350, 600, 530) einen Körperabschnitt (30, 76, 104, 684, 220, 270, 320, 370, 620, 354) und einen Pufferelementhalter (12, 52, 62, 608, 72, 106, 686, 230, 280, 330, 380, 630, 536) aufweist, der vom Körperabschnitt (30, 76, 104, 684, 220, 270, 320, 370, 620, 354) radial hervorsteht, um zu verhindern, dass der Stent (10, 600, 150, 540) sich distal bewegt, wenn die Hülse (44, 520) während des Zuführens des Stents (10, 600, 150, 540) zurückgezogen wird;
**dadurch gekennzeichnet, dass** das Verfahren aufweist:
einen Schritt zum Ineingriffbringen einer Zelle (22, 601, 148, 542, 544) des Stents (10, 600, 150, 540) mit dem Pufferelementhalter (12, 52, 62, 608, 72, 106, 686, 230, 280, 330, 380, 630, 536) ohne einen Durchmesser des Stents (10, 600, 150, 540) zu vermindern durch zwei Paddel (20, 602, 152, 672), die in der Zelle des Stents (10, 600, 150, 540, 850) vorgesehen sind und sich biegen, um sich um den Pufferelementhalter (12, 52, 62, 608, 72, 106, 686, 230, 280, 330, 380, 630) zu erstrecken, wenn der Stent (10, 600, 150) in einer axialen Richtung (A) gegen den Pufferelementhalter (12, 52, 62, 608, 72, 106, 686, 230, 280, 330, 380, 630) gedrückt wird, wobei die Zelle eine teilweise offene Zelle ist, oder durch Dehnen des Pufferelementhalters (536, 810), so dass er mit der Zelle (542, 544) des Stents (540) in Eingriff kommt.

2. Verfahren nach Anspruch 1, wobei der Pufferelementhalter (12, 52, 62, 608, 72, 106, 686, 230, 280, 330, 380, 630) eine vom Körperabschnitt (30, 76, 104, 684, 220, 270, 320, 370, 620, 354) radial hervorstehende Lasche aufweist.

3. Verfahren nach Anspruch 1, wobei der Pufferelementhalter (12, 52, 62, 608, 72, 106, 686, 230, 280, 330, 380, 630) einen Haken, eine Zacke oder einen Widerhaken aufweist.

4. Verfahren nach Anspruch 1, wobei der Pufferelementhalter (12, 52, 62, 608, 72, 106, 686, 230, 280, 330, 380, 630) dazu geeignet ist, sich zu biegen, um mit der teilweise offenen Zelle (22, 601, 148) in Eingriff oder davon außer Eingriff zu kommen.

5. Verfahren nach Anspruch 1, wobei der Pufferelementhalter (12, 52, 62, 608, 72, 106, 686, 230, 280, 330, 380, 630) ein Wachs aufweist.

6. Verfahren nach einem der Ansprüche 1 bis 4, wobei die zwei Paddel (20, 602, 152, 672) jeweils einen Kopf (26, 604, 156) und einen Schenkel (28, 606, 158) aufweisen.

7. Verfahren nach Anspruch 6, wobei die Paddel (20, 602, 152, 672) aus einem strahlenundurchlässigen Material hergestellt sind.

8. Verfahren nach Anspruch 6 oder 7, wobei das Biegen der Paddel (20, 602, 152, 672) um den Pufferelementhalter (12, 52, 62, 608, 72, 106, 686, 230, 280, 330, 380, 630) einen Eingriff zwischen der teilweise offenen Zelle (22, 601, 148) und dem Pufferelementhalter (12, 52, 62, 608, 72, 106, 686, 230, 280, 330, 380, 630) ermöglicht.

9. Verfahren nach Anspruch 1, wobei der Pufferelementhalter (536) des Katheters derart konfiguriert ist, dass er sich von einer ersten Größe auf eine zweite Größe dehnt.

10. Verfahren nach Anspruch 1 oder 9, wobei der Pufferelementhalter (536) des Katheters aus einem Formgedächtnismaterial hergestellt ist.

11. Stentzufuhrsystem mit:
einem Katheter mit einem inneren Katheter (42, 520);
einer Hülse (44, 510), die den Katheter mindestens teilweise umschließt;
einem zwischen dem Katheter und der Hülse angeordneten Stent (10, 600, 150, 540)
einem Pufferelement (14, 50, 60, 610, 70, 100, 680, 200, 250, 300, 350, 600, 530), das auf den inneren Katheter (42, 520) geladen ist, wobei das Pufferelement (14, 50, 60, 610, 70, 100, 680, 200, 250, 300, 350, 600, 530) einen Körperabschnitt (30, 76, 104, 684, 220, 270, 320, 370, 620, 354) und einen Pufferelementhalter (12, 52, 62, 608, 72, 106, 686, 230, 280, 330, 380, 630, 536) aufweist, der vom Körperabschnitt (30, 76, 104, 684, 220, 270, 320, 370, 620, 354) radial hervorsteht,
wobei der Pufferelementhalter (12, 52, 62, 608, 72, 106, 686, 230, 280, 330, 380, 630, 536) dafür konfiguriert ist, mit einer Zelle (22, 601, 148, 542, 544) des Stents (10, 600, 150, 540) in Eingriff zu kommen, um den Stent (10, 600, 150, 540) bezüglich des inneren Katheters unidirektional zu positionieren;
**dadurch gekennzeichnet, dass**
das Stentzufuhrsystem dafür konfiguriert ist, eine Zelle (22, 601, 148, 542) des Stents (10, 600, 150, 540) mit dem Pufferelementhalter (12, 52, 62, 608, 72, 106, 686, 230, 280, 330, 380, 630, 536) in Eingriff zu bringen, ohne einen Durchmesser des Stents (10, 600, 150, 540) zu vermindern durch zwei Paddel (20, 602, 152, 672), die in der Zelle des Stents (10, 600, 150, 540) vorgesehen sind und sich biegen, um sich um den Pufferelementhalter (12, 52, 62, 608, 72, 106, 686, 230, 280, 330, 380, 630, 536) zu erstrecken, wenn der Stent (10, 600, 150) in der axialen Richtung (A) gegen den Pufferelementhalter (12, 52, 62, 608, 72, 106, 686, 230, 280, 330, 380, 630) gedrückt wird, wobei die Zelle eine teilweise offene Zelle ist, oder durch einen sich dehnenden Pufferelementhalter (536), der mit einer Zelle (542, 544) des Stents (540) in Eingriff kommt.

12. Stentzufuhrsystem nach Anspruch 11, wobei der Pufferelementhalter (12, 52, 62, 608, 72, 106, 686, 230, 280, 330, 380, 630) eine Lasche aufweist, die vom Körperabschnitt (30, 76, 104, 684, 220, 270, 320, 370, 620, 354) radial hervorsteht.

13. Stentzufuhrsystem nach Anspruch 12, wobei der Pufferelementhalter (12, 52, 62, 608, 72, 106, 686, 230, 280, 330, 380, 630) einen Haken, eine Zacke oder einen Widerhaken aufweist.

14. Stentzufuhrsystem nach Anspruch 13, wobei der Pufferelementhalter (12, 52, 62, 608, 72, 106, 686, 230, 280, 330, 380, 630) dazu geeignet ist, sich zu biegen, um mit der teilweise offenen Zelle (22, 601, 148) in Eingriff oder davon außer Eingriff zu kommen.

15. Stentzufuhrsystem nach Anspruch 14, wobei der Pufferelementhalter (12, 52, 62, 608, 72, 106, 686, 230, 280, 330, 380, 630) ein Wachs aufweist.

16. Stentzufuhrsystem nach einem der Ansprüche 11 bis 14, wobei die zwei Paddel (20, 602, 152, 672) jeweils einen Kopf (26, 604, 156) und einen Schenkel (28, 606, 158) aufweisen.

17. Stentzufuhrsystem nach Anspruch 16, wobei die Paddel (20, 602, 152, 672) aus einem strahlenundurchlässigen Material hergestellt sind.

18. Stentzufuhrsystem nach Anspruch 16 oder 17, wobei das Biegen der Paddel (20, 602, 152, 672) um den Pufferelementhalter (12, 52, 62, 608, 72, 106, 686, 230, 280, 330, 380, 630) einen Eingriff zwischen der teilweise offenen Zelle (22, 601, 148, 712) und dem Pufferelementhalter (12, 52, 62, 608, 72, 106, 686, 230, 280, 330, 380, 630) ermöglicht.

19. Stentzufuhrsystem nach Anspruch 11, wobei der Pufferelementhalter (536) des Katheters derart konfiguriert ist, dass er sich von einer ersten Größe auf eine zweite Größe dehnt.

20. Stentzufuhrsystem nach Anspruch 11 oder 19, wobei der Pufferelementhalter (536) des Katheters aus einem Formgedächtnismaterial hergestellt ist.

## Revendications

1. Procédé de positionnement d'un stent (10, 600, 150, 540, 850) sur un système de délivrance, le système de délivrance comprenant
un cathéter interne (42, 520),
une gaine (44, 510), et
un butoir (14, 50, 60, 610, 70, 100, 680, 200, 250, 300, 350, 600, 530) chargé sur le cathéter interne (42, 520), le butoir (14, 50, 60, 610, 70, 100, 680, 200, 250, 300, 350, 600, 530) incluant une partie formant corps (30, 76, 104, 684, 220, 270, 320, 370, 620, 354) et un dispositif de retenue de butoir (12, 52, 62, 608, 72, 106, 686, 230, 280, 330, 380, 630, 536) qui fait saillie radialement de la partie formant corps (30, 76, 104, 684, 220, 270, 320, 370, 620, 354) pour empêcher le stent (10, 600, 150, 540) de se déplacer distalement quand la gaine (44, 520) est retirée pendant la délivrance du stent (10, 600, 150, 540),
**caractérisé en ce que** le procédé inclut l'étape d'entrecroisement d'une cellule (22, 601, 148, 542, 544) du stent (10, 600, 150, 540) avec le dispositif de retenue de butoir (12, 52, 62, 608, 72, 106, 686, 230, 280, 330, 380, 630, 536) est accomplie sans réduire un diamètre du stent (10, 600, 150, 540) par deux pales (20, 602, 152, 672) incluses dans la cellule du stent (10, 600, 150, 540, 850) qui fléchissent pour s'étendre autour du dispositif de retenue de butoir (12, 52, 62, 608, 72, 106, 686, 230, 280, 330, 380, 630) quand le stent (10, 600, 150) est poussé dans une direction axiale (A) contre le dispositif de retenue de butoir (12, 52, 62, 608, 72, 106, 686, 230, 280, 330, 380, 630) où la cellule est une cellule partiellement ouverte, ou bien par expansion du dispositif de retenue de butoir (536, 810) pour s'entrecroiser avec la cellule (542, 544) du stent (540).

2. Procédé selon la revendication 1, où le dispositif de retenue de butoir (12, 52, 62, 608, 72, 106, 686, 230, 280, 330, 380, 630) comprend un volet qui fait saillie radialement de la partie formant corps (30, 76, 104, 684, 220, 270, 320, 370, 620, 354).

3. Procédé selon la revendication 1, où le dispositif de retenue de butoir (12, 52, 62, 608, 72, 106, 686, 230, 280, 330, 380, 630) comprend un crochet, une pointe ou un picot.

4. Procédé selon la revendication 1, où le dispositif de retenue de butoir (12, 52, 62, 608, 72, 106, 686, 230, 280, 330, 380, 630) est capable de fléchir pour s'entrecroiser ou se désengager de la cellule partiellement ouverte (22, 601, 148).

5. Procédé selon la revendication 1, où le dispositif de retenue de butoir (12, 52, 62, 608, 72, 106, 686, 230, 280, 330, 380, 630) comprend une cire.

6. Procédé selon l'une des revendications 1 à 4, où les deux pales (20, 602, 152, 672) ont chacune une tête (26, 604, 156) et une jambe (28, 606, 158).

7. Procédé selon la revendication 6, où les pales (20, 602, 152, 672) sont formées d'un matériau radio-opaque.

8. Procédé selon la revendication 6 ou 7, où la flexion des pales (20, 602, 152, 672) autour du dispositif de retenue de butoir (12, 52, 62, 608, 72, 106, 686, 230, 280, 330, 380, 630) permet l'entrecroisement entre la cellule partiellement ouverte (22, 601, 148) et le dispositif de retenue de butoir (12, 52, 62, 608, 72, 106, 686, 230, 280, 330, 380, 630).

9. Procédé selon la revendication 1, où le dispositif de retenue de butoir (536) du cathéter est configuré pour s'expanser depuis une première dimension jusqu'à une seconde dimension.

10. Procédé selon la revendication 1 ou 9, où le dispositif de retenue de butoir (536) du cathéter est formé d'un matériau à mémoire de forme.

11. Système de délivrance de stent, comprenant :
un cathéter ayant un cathéter interne (42, 520),
une gaine (44, 510) entourant au moins partiellement le cathéter,
un stent (10, 600, 150, 540) disposé entre le cathéter et la gaine,
un butoir (14, 50, 60, 610, 70, 100, 680, 200, 250, 300, 350, 600, 530) chargé sur le cathéter interne (42, 520), le butoir (14, 50, 60, 610, 70, 100, 680, 200, 250, 300, 350, 600, 530) incluant une partie formant corps (30, 76, 104, 684, 220, 270, 320, 370, 620, 354) et un dispositif de retenue de butoir (12, 52, 62, 608, 72, 106, 686, 230, 280, 330, 380, 630, 536) qui fait saillie radialement de la partie formant corps (30, 76, 104, 684, 220, 270, 320, 370, 620, 354),
où le dispositif de retenue de butoir (12, 52, 62, 608, 72, 106, 686, 230, 280, 330, 380, 630, 536) est configuré pour s'entrecroiser avec une cellule (22, 601, 148, 542, 544) du stent (10, 600, 150, 540) pour positionner le stent (10, 600, 150, 540) de manière unidirectionnelle par rapport au cathéter interne **caractérisé en ce que** le système de délivrance de stent est configuré pour entrecroiser une cellule (22, 601, 148, 542) du stent (10, 600, 150, 540) avec le dispositif de retenue de butoir (12, 52, 62, 608, 72, 106, 686, 230, 280, 330, 380, 630, 536) sans réduire un diamètre du stent (10, 600, 150, 540) par deux pales (20, 602, 152, 672) incluses dans la cellule du stent (10, 600, 150, 540) qui fléchissent pour s'étendre autour du dispositif de retenue de butoir (12, 52, 62, 608, 72, 106, 686, 230, 280, 330, 380, 630) quand le stent (10, 600, 150) est poussé dans une direction axiale (A) contre le dispositif de retenue de butoir (12, 52, 62, 608, 72, 106, 686, 230, 280, 330, 380, 630) où la cellule est une cellule partiellement ouverte, ou bien par un dispositif de retenue de butoir qui s'expanse (536) qui s'entrecroise avec une cellule (542, 544) du stent (540).

12. Système de délivrance de stent selon la revendication 11, où le dispositif de retenue de butoir (12, 52, 62, 608, 72, 106, 686, 230, 280, 330, 380, 630) comprend un volet qui fait saillie radialement de la partie formant corps (30, 76, 104, 684, 220, 270, 320, 370, 620, 354).

13. Système de délivrance de stent selon la revendication 12, où le dispositif de retenue de butoir (12, 52, 62, 608, 72, 106, 686, 230, 280, 330, 380, 630) comprend un crochet, une pointe ou un picot.

14. Système de délivrance de stent selon la revendication 13, où le dispositif de retenue de butoir (12, 52, 62, 608, 72, 106, 686, 230, 280, 330, 380, 630) est capable de fléchir pour s'entrecroiser ou se désengager de la cellule partiellement ouverte (22, 601, 148).

15. Système de délivrance de stent selon la revendication 14, où le dispositif de retenue de butoir (12, 52, 62, 608, 72, 106, 686, 230, 280, 330, 380, 630) comprend une cire.

16. Système de délivrance de stent selon l'une des revendications 11 à 14, où les deux pales (20, 602, 152, 672) ont chacune une tête (26, 604, 156) et une jambe (28, 606, 158).

17. Système de délivrance de stent selon la revendication 16, où les pales (20, 602, 152, 672) sont formées d'un matériau radio-opaque.

18. Système de délivrance de stent selon la revendication 16 ou 17, où la flexion des pales (20, 602, 152, 672) autour du dispositif de retenue de butoir (12, 52, 62, 608, 72, 106, 686, 230, 280, 330, 380, 630) permet un entrecroisement entre la cellule partiellement ouverte (22, 601, 148, 712) et le dispositif de retenue de butoir (12, 52, 62, 608, 72, 106, 686, 230, 280, 330, 380, 630).

19. Système de délivrance de stent selon la revendication 11, où le dispositif de retenue de butoir (536) du cathéter est configuré pour s'expanser depuis une première dimension jusqu'à une seconde dimension.

20. Système de délivrance de stent selon la revendication 11 ou 19, où le dispositif de retenue de butoir (536) du cathéter est formé d'un matériau à mémoire de forme.
